# EUROPÄISCHE PATENTSCHRIFT

(11) **EP 1 683 532 B1**
(45) Veröffentlichungstag und Bekanntmachung des Hinweises auf die Patenterteilung: **15.06.2011**
(21) Anmeldenummer: 06005573.8
(22) Anmeldetag: 08.12.2000
(51) Int. Cl.: A61M 1/16

(54) **Vorrichtung zur Durchführung einer medizinischen Behandlung mit einer als formgebender Schale ausgebildeter Aufnahmeeinheit für einen Folienbeutel**
Medical treatment device comprising a receiving unit in the form of a jacket for a plastic bag
Dispositif de traitement médical comprenant une unité de réception en forme d'enveloppe pour sacs plastiques

(30) Priorität: 08.12.1999 DE 19959230
(43) Veröffentlichungstag der Anmeldung: 26.07.2006
(62) Teilanmeldung aus: 00979676.4
(73) Patentinhaber: Fresenius Medical Care Deutschland GmbH, 61352 Bad Homburg v.d.H. (DE)
(72) Erfinder: Hilgers, Peter, 97453 Schonungen (DE); Brandl, Matthias, 97631 Bad Königshofen (DE)
(74) Vertreter: Luderschmidt, Wolfgang

(56) Entgegenhaltungen:
- DE-A- 19 633 657
- FR-A- 2 749 763
- US-A- 5 364 385
- US-A- 5 607 082

## Beschreibung

Die Erfindung betrifft eine Vorrichtung zur Durchführung einer medizinischen Behandlung unter Verwendung einer Flüssigkeit.

Hämodialysegeräte sind in verschiedenen Ausführungen bekannt. Der Stoffaustausch zwischen dem Blut und der Dialysierflüssigkeit findet in einem Dialysator statt, der einen ersten Strömungsweg für das Blut und einen zweiten Strömungsweg für die Dialysierflüssigkeit aufweist, wobei beide Strömungswege durch eine semipermeable Membran voneinander getrennt sind. Der erste Strömungsweg ist Bestandteil eines extrakorporalen Blutkreislaufs mit einer Zuführleitung und einer Rückführleitung für das Blut sowie ggf. einer den Blutfluß unterstützenden Pumpe. Der zweite Strömungsweg ist mit Einrichtungen zum Zuführen und Abführen der Dialysierflüssigkeit verbunden. Neben den sogenannten Single-Pass-Systemen, bei denen die kontinuierlich zugeführte Dialysierflüssigkeit nur einmal den Dialysator passiert und dann verworfen wird, sind auch sogenannte Batch-Systeme bekannt. Die DE 31 15 665 C2 beschreibt ein derartiges Hämodialysiergerät, das mit einem gegen die Atmosphäre abgeschlossenen, volumenstarren Behälter arbeitet, der vor Beginn der Behandlung vollständig mit frischer Dialysierflüssigkeit gefüllt wird. Während des Betriebs wird Flüssigkeit aus dem Behälter durch den Dialysator gepumpt und die verbrauchte Flüssigkeit wird wieder in den Behälter zurückgeleitet. Aufgrund des konstanten Volumens des gesamten mit der Dialysierflüssigkeit gefüllten Systems kann eine Ultrafiltration nur dann erfolgen, wenn dem System Flüssigkeit entzogen wird. Eine Vermischung von frischer und verbrauchter Dialysierflüssigkeit wird bei dem bekannten Härnodialysegerät dadurch vermieden, daß die Entnahme der Dialysierflüssigkeit im oberen Bereich des Behälters erfolgt, während die Rückführung im unteren Behälterbereich stattfindet. Die Unterschichtung der frischen Dialysierflüssigkeit mit der verbrauchten Dialysierflüssigkeit bleibt durch die Aufrechterhaltung eines vertikalen Temperaturgefälles in dem Behälter von oben nach unten stabil.

Der Behälter besteht aus Glas, das weitgehend resistent gegen in Betracht kommende Chemikalien, gut zu reinigen und physiologisch unbedenklich ist. Nachteilig ist jedoch, daß der Glasbehälter verhältnismäßig teuer in der Herstellung und relativ schwierig zu reinigen ist.

Aus der DE 198 25 158 C1 ist ein als Folienbeutel ausgebildetes Disposable bekannt, daß zusammen mit einer formgebenden Schale den in der Herstellung relativ teueren Glasbehälter der Hämodialysevorrichtung ersetzen kann, die in der eingangs erwähnten DE 31 55 665 C2 beschrieben ist. Das Disposable besteht aus zwei drehkegelförmigen Teilstücken, die an ihrem umlaufenden Rand zu einem Doppelkegel miteinander verschweißt sind. Der besondere Vorteil dieser Formgebung liegt daran, daß sich das Disposable aus handelsüblichen (ebenen) Folien herstellen läßt. Eine räumliche Formgebung, wie beispielsweise bei Einmal-Handschuhen, ist hierzu nicht erforderlich.

Der Glasbehälter kann nur dann durch den Folienbeutel ersetzt werden, wenn die Volumenkonstanz, Keimfreiheit und Flüssigkeitsunterschichtung gewährleistet ist.

Um die Volumenkonstanz sicherzustellen, muß sich der Beutel während des Füllens reproduzierbar und vollständig in der druckstabilen Schale mit definiertem Volumen entfalten können. Lufteinschlüsse zwischen Beutel und Schale, die durch Entfaltungen oder zu überbrückende Zwischenräume entstehen, müssen weitgehend vermieden werden. Durch die Geometrie bedingte Falten können nur dann akzeptiert werden, sofern sie reproduzierbar sind und das von ihnen eingeschlossene Volumen im Vergleich zum Gesamtvolumen vernachlässigbar ist. Zur Gewährleistung der Keimfreiheit darf der Innenraum des Beutels während der Dialysevorbereitung nicht geöffnet und während des Füllens nur in Kontakt mit der einströmenden Flüssigkeit kommen. Zur Flüssigkeitsunterschichtung muß die Form des Beutels eine klare Trennung zwischen frischem und verbrauchtem Dialysat unterstützen. Darüber hinaus soll der Beutel einfach zu handhaben und kostengünstig herzustellen sein.

Die FR 2 749 763 beschreibt ein Verfahren zur Herstellung eines Salzkonzentrates für die Hämodialyse. Das Salzkonzentrat wird in einem Beutel bereitgestellt, der aus einer Schlauchfolie gefaltet wird. Es handelt sich um einen Seitenfaltbeutel, der sich zu einer rechteckförmigen Packung aufklappen lässt.

Die US-A-5 607 082 beschreibt einen Tank für eine Sprühpistole mit einem kastenförmigen Außenbehälter, der einen Innenbehälter aufnimmt. Der Innenbehälter weist eine feste Rückwand auf und ist harmonikaartig gefaltet.

Aus der DE 9417416 U1 ist ein Sekretauffangbeutel bekannt, der harmonikaartig zusammenfaltbar ist.

Die DE 196 33 657 A beschreibt eine Blutbehandlungsvorrichtung, die sowohl für den Einnadel-Betrieb als auch Zweinadel-Betrieb ausgelegt ist. Die Dialysevorrichtung verfügt über zwei Äufnahmeeinheiten, in die Blutspeicherbeutel eingelegt werden. Die beiden Aufnahmeeinheiten für die Blutspeicherbeutel weisen jeweils zwei starre Druckkörper auf, von denen der eine ortsfest angeordnet und der andere mittels eines Federelements vorgespannt ist, das an einer Grundplatte befestigt ist. Die plattenförmigen Druckkörper der Aufnahmeeinheiten sind auf beiden Seiten der Blutbeutel angeordnet, so dass sie die Blutbeutel nur von der Seite erfassen, nicht aber von allen Seiten umschließen.

Der Erfindung liegt die Aufgabe zugrunde, eine medizinische Behandlungsvorrichtung für ein einfach zu handhabendes und kostengünstig herstellbares Disposable zu schaffen, das die erforderliche Volumenkonstanz, Keimfreiheit und Flüssigkeitsunterschichtung gewährleistet.

Die Lösung dieser Aufgabe erfolgt mit dem Gegenstand des Patentanspruchs 1.

Das Disposable zur Verwendung in der erfindungsgemäßen Behandlungsvorrichtung wird aus zwei übereinander liegenden Flachfolien oder einer Schlauchfolie hergestellt, wobei die ein oder zwei Längsschweißnähte zum Versiegeln der übereinander liegenden Flachfolien bei der Verwendung einer Schlauchfolie entfallen können.

An dem in der Gebrauchslage oberen Ende ist der Folienbeutel bzw. die Schlauchfolie mit einer ersten Schweißnaht und an dem unteren Ende mit einer zweiten Schweißnaht verschlossen. Beide Schweißnähte haben vorzugsweise einen nach außen gekrümmten Verlauf. Der entfaltete Folienbeutel hat damit vorzugsweise einen zylindrischen Abschnitt, an den sich ein nach außen gewölbter oberer und ein nach außen gewölbter unterer Abschnitt anschließen. Das gleiche gilt für die Schlauchfolie.

Unter nach außen gekrümmten Schweißnähten sind aber auch Schweißnähte zu verstehen, die sich aus mehreren geraden Abschnitten zusammensetzten. So hat es sich in der Praxis als besonders vorteilhaft erwiesen, anstelle von bogenförmigen Schweißnähten Schweißnähte mit geraden Abschnitten vorzusehen, die den Verlauf der Bogenschweißnähte approximieren. Derartige Schweißnähte lassen sich produktionstechnisch besonders einfach anfertigen.

Die medizinische Behandlungsvorrichtung verfügt über eine als formgebende Schale ausgebildete Aufnahmeeinheit für das Disposable. Die formgebende Schale weist eine mittlere zylindrische Anlagefläche auf, an die sich eine nach außen gewölbte obere Anlagefläche und eine nach außen gewölbte untere Anlagefläche anschließen. Die Schale zeichnet sich somit durch eine hohe Druckstabilität aus.

Für die medizinische Behandlung wird das Disposable in die formgebende Schale eingelegt. Da sich die gewölbten Abschlüsse des Zylinders durch ebene Flächen nicht exakt abwickeln lassen, ist zwar eine Faltenbildung des Folienbeutels bzw. der Schlauchfolie in den Abschlüssen unvermeidbar, in der Praxis hat sich jedoch gezeigt, daß das von den Falten in den Abschlüssen eingeschlossene Volumen im Vergleich zum Gesamtvolumen relativ gering ist.

Der Folienbeutel bzw. die Schlauchfolie ist parallel der in der Gebrauchslage vertikalen Längsachse mehrfach gefaltet. Vorzugsweise ist der Folienbeutel bzw. die Schlauchfolie mehrfach in gleich große Abschnitte, vorteilhafterweise harmonikaartig gefaltet, wobei es für einen symmetrischen Aufbau vorteilhaft ist, eine ungerade Anzahl von Faltabschnitten zu wählen. Zum Einführen des Disposables ist die formgebende Schale in der oberen Anlagefläche mit einer Ausnehmung versehen. Da der gefaltete Folienbeutel bzw. die Schlauchfolie einen flachen Streifen bildet, können die Abmessungen der Ausnehmung gering sein. Folglich sind auch die auf die Ausnehmung durch den Systemdruck wirkenden Kräfte gering, so daß die Schale sehr druckstabil und der Verschluß der Ausnehmung vereinfacht ist. Da aufwendige Mechanismen beispielsweise zum Aufklappen der Schale oder dgl. nicht erforderlich sind, ist auch die Herstellung der Schale relativ einfach.

Das Disposable stellt eine aufgrund der einfachen geometrischen Form kostengünstig herzustellende Anordnung dar, die sich einfach handhaben läßt. Die Beutelform unterstützt die reproduzierbare Entfaltung. Neben der Optimierung des Ausbreitungsverhaltens hat die Faltung auch den Vorteil, daß der Beutel bei der Lagerung und dem Transport weniger Raum einnimmt.

Um das Disposable als flachen Streifen bereitstellen zu können, sind die Faltungen zweckmäßigerweise mit Verbindungselementen, beispielsweise Nieten, Klammern oder dgl. fixiert, die vorzugsweise an den Folienenden angeordnet sind. Es können aber auch Schweißnähte senkrecht quer zur Längsachse vorgesehen sein.

In einer bevorzugten Ausführungsform des Disposables ist an der ersten Schweißnaht ein Anschlußteil mit der Beutelfolie verschweißt, der mindestens einen Anschluß zum Zuführen von Flüssigkeit und einen Anschluß zum Abführen von Flüssigkeit aufweist. An dem Anschluß zum Zuführen von Flüssigkeit ist eine sich in das Beutelinnere bis zu der zweiten Schweißnaht erstreckende Schlauchleitung angeschlossen. Dadurch wird erreicht, daß Flüssigkeit, beispielsweise verbrauchte Dialysierflüssigkeit, der unteren Hälfte zugeführt und aus der oberen Hälfte des Disposables Flüssigkeit, beispielsweise frische Dialysierflüssigkeit, abgezogen werden kann.

Zur Fixierung der Schlauchleitung in dem Disposable kann an der zweiten Schweißnaht ein Fixierungsstück vorgesehen sein, an dem die Schlauchleitung befestigt ist.

Der Folienbeutel bzw. die Schlauchfolie besteht vorzugsweise aus einem Polyethylen-Basismaterial, das einseitig mit einer Polyamid-Siegelschicht versehen ist.

Bei einer weiteren bevorzugten Ausführungsform der medizinischen Behandlungsvorrichtung kann das untere Ende des Disposables in der formgebenden Schale fixiert werden. Hierzu ist die Schale im Zentrum der unteren Anlagefläche mit einer zweiten Ausnehmung versehen, in die eine Fixierungseinrichtung zur lösbaren Befestigung des unteren Endes des Disposables paßgenau eingesetzt werden kann. Die Ausnehmung hat vorzugsweise einen sich nach unten verjüngenden Querschnitt, so daß eine besonders einfache Positionierung der entsprechend geformten Fixierungseinrichtung möglich ist.

Die Fixierungseinrichtung ist vorzugsweise in der Schale arretierbar. Zur Fixierung können beispielsweise eine Magnetkupplung oder auch ein Schloß oder dergleichen vorgesehen sein. Allein entscheidend ist, daß die Arretierung zum Austausch des Disposables von Hand leicht lösbar ist.

Zur Befestigung des oberen Endes des Disposables ist vorzugsweise eine weitere Fixierungseinrichtung vorgesehen, die in die Ausnehmung zum Einführen des Disposables in die Schale paßgenau einsetzbar ist. Diese Fixierungseinrichtung dient also gleichsam dem Verschluß der Schale.

Die zweite Fixierungseinrichtung ist vorzugsweise längsverschiebbar an einer Halteeinrichtung befestigt. Beim Befüllen des Disposables verringert sich der Abstand zwischen seinem oberen und unteren Ende, wobei die längsverschiebbare Fixierungseinrichtung die Schale verschließt. Da die Fixierungseinrichtung außerhalb der Schale angeordnet ist, kann das obere Ende des Disposables leicht befestigt werden.

Die Faltungen geben eine parallel und eine senkrecht zur Flächennormalen der Faltenabschnitte gerichtete Orientierung vor. Diese Orientierungen sind durch die Mehrlagigkeit der Faltenabschnitte versteift, wodurch sie beim Einführen des Disposables in die Schale raumfest erhalten bleiben. Die erste und zweite Fixierungseinrichtung stellen sicher, daß der Beutel beim Befüllen straff gehalten wird. Dadurch ist gewährleistet, daß der Füllvorgang unter eindeutig definierten Bedingungen stattfindet und genau reproduzierbar ist.

Der Verlauf der ersten und zweiten Schweißnaht wird vorzugsweise so gewählt, daß die Faltenbildung in den Zylinderabschlüssen minimiert und reproduzierbar ist. Dies kann insbesondere mit einer bogenförmigen Schweißnaht erreicht werden, die nur Falten senkrecht zur Naht erzeugt. Wenn das Disposable befüllt ist, sind bei einer bogenförmigen Schweißnaht die auftretenden Spannungen verhältnismäßig gering.

In Versuchen hat sich gezeigt, daß sich das Disposable insbesondere dann leicht entfalten und an die Anlageflächen der Schale anlegen kann, wenn der Abstand zwischen den Enden der ersten und zweiten Schweißnaht auf einer der beiden Längsseiten des flachliegenden Folienbeutels bzw. der Schlauchfolie größer ist, als die Länge der zylindrischen Anlagefläche der formgebenden Schale. Der Abstand sollte vorzugsweise um einen Betrag größer sein, der mindestens doppelt so groß, vorzugsweise dreimal so groß wie die Breite der Falten des Folienbeutels bzw. der Schlauchfolie ist.

Eine weitere Verbesserung wird dadurch erzielt, wenn gleichzeitig der Abstand zwischen dem Scheitelpunkt der ersten bzw. zweiten Schweißnaht und dem Mittelpunkt der durch deren Enden verlaufenden Geraden bei flachliegendem Folienbeutel bzw. Schlauchfolie kleiner als die Tiefe der gewölbten Anlagefläche der formgebenden Schale ist. Damit wird die Krümmung der Bogenschweißnähte geringer, wodurch sich diese in ihren Randbereichen auch durch gerade Schweißnähte ersetzen lassen.

Das Disposable findet vorzugsweise in einer Hämodialysevorrichtung Verwendung. Es kann aber auch ähnlich wie in der DE 198 25 158 C1 beschrieben in einer Peritonealdialyse-Vorrichtung zur Bereitstellung von Peritonealdialyse-Lösung verwendet werden.

Im folgenden werden einzelne Ausführungsbeispiele der Erfindung unter Bezugnahme auf die Zeichnungen näher erläutert.

Es zeigen:
- Figur 1: eine vereinfachte Darstellung des flachliegenden Disposables in der Draufsicht,
- Figur 2: das gefaltete Disposable von Figur 1,
- Figur 3: die harmonikaartige Faltung des Disposables,
- Figur 4: eine vereinfachte Darstellung der Aufnahmeeinheit für das Disposable von Figur 1,
- Figur 5: den Ausschnitt A von Figur 4 in vergrößerter perspektivischer Darstellung,
- Figur 6: den Ausschnitt B von Figur 4 in vergrößerter perspektivischer Darstellung,
- Figur 7: die Aufnahmeeinheit von Figur 4, in die das Disposable von Figur 1 eingelegt ist,
- Figur 8: die an die Aufnahmeeinheit angepaßten Schweißnähte des Disposables von Figur 1,
- Figur 9: eine Hämodialysevorrichtung mit der Aufnahmeeinheit von Figur 4 und dem Disposable von Figur 1 in vereinfachter Darstellung und
- Figur 10: ein weiteres Ausführungsbeispiel des Disposables in der Draufsicht.

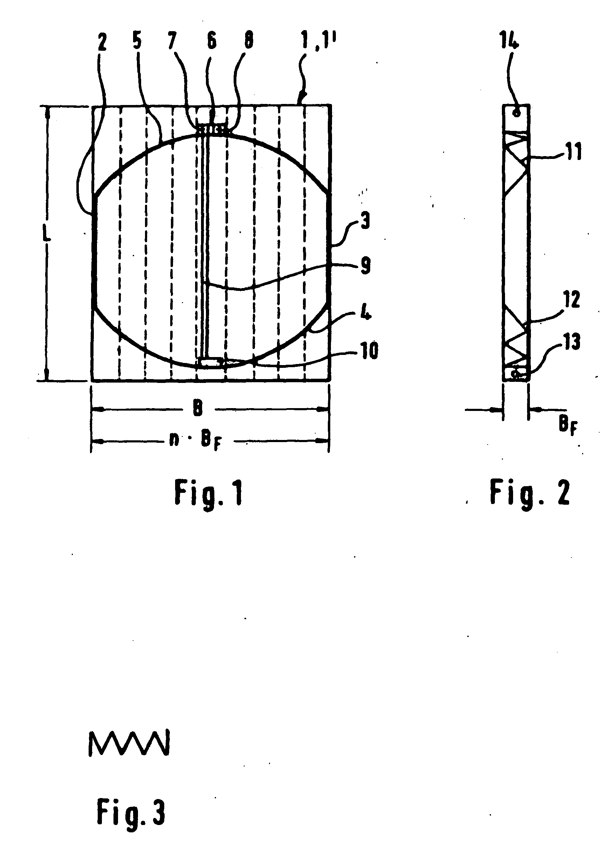

Figur 1 zeigt die Draufsicht auf das flachliegende Disposable in vereinfachter Darstellung. Das Disposable weist zwei rechteckförmige Folien 1, 1' mit der Länge L und der Breite B auf, die deckungsgleich übereinander liegen und an den Längsseiten mit Längsschweißnähten 2, 3 und an den Enden mit nach außen gekrümmten Bogenschweißnähten 4, 5 versiegelt sind. Als Folienmaterial wird eine Flachfolie verwendet, die aus Polyethylen (PE) als Basismaterial besteht, das einseitig mit einer Polyamid (PA) -Siegelschicht versehen ist. Die Dicke der Folie beträgt 100 µm.

Anstelle der übereinander liegenden Flachfolien kann auch ein Folienschlauch verwendet werden, der an seinen Enden mit den Bogenschweißnähten verschlossen wird. Es ist aber auch möglich eine Flachfolie zu verwenden, die einseitig gefaltet, nur an einer Längsseite mit einer Längssschweißnaht versiegelt und an ihren Enden mit den Bogenschweißnähten verschlossen wird.

Das Disposable verfügt über einen in Figur 1 nur andeutungsweise dargestellten Anschlußteil 6, der beispielsweise nach Art eines Schiffchens ausgebildet und mit den Flachfolien versiegelt ist. Der Anschlußteil 6 weist einen Anschluß 7 zum Zuführen und einen Anschluß 8 zum Abführen von Flüssigkeit auf. An den Anschlüssen 7 und 8 werden die Schlauchleitungen der medizinischen Behandlungsvorrichtung angeschlossen.

Der Anschlußteil 6 ist am Scheitelpunkt der in der Gebrauchslage des Disposables oberen Bogenschweißnaht 5 angeordnet. An dem Anschluß 7 zum Zuführen von Flüssigkeit ist eine sich in das Beutelinnere bis zu der unteren Bogenschweißnaht 4 erstreckende Schlauchleitung 9 angeschlossen, deren freies Ende an einem Fixierungsstück 10 befestigt ist, das am Scheitelpunkt der unteren Schweißnaht 4 mit den Flachfolien 1, 1' versiegelt ist. Der flachliegende Folienbeutel wird parallel zu seiner Längsachse mehrfach in gleich große Abschnitte harmonikaartig gefaltet. Die Breite B_{F} der Faltabschnitte berechnet sich aus dem Quotienten der Breite B der Flachfolien und der Anzahl n der Faltabschnitte. Für einen symmetrischen Aufbau ist es zweckmäßig, eine ungerade Anzahl von Faltabschnitten zu wählen (z. B. n = 9).

Figur 2 zeigt den harmonikaartig gefalteten Folienbeutel von Figur 1 in der Draufsicht. Die gefalteten Bogenschweißnähte an den Enden des Folienbeutels sind mit den Bezugszeichen 11, 12 versehen. Figur 3 zeigt die harmonikaartige Faltung des Folienbeutels.

Zur Fixierung der Faltungen sind in Figur 2 nur andeutungsweise dargestellte Verbindungselemente 13, 14 vorgesehen, die an den über die Bogenschweißnähte 4, 5 überstehenden Enden des Folienbeutels angeordnet sind. Die Verbindungselemente sind Nieten, mit denen der Folienstapel als flacher Streifen fest zusammengehalten wird.

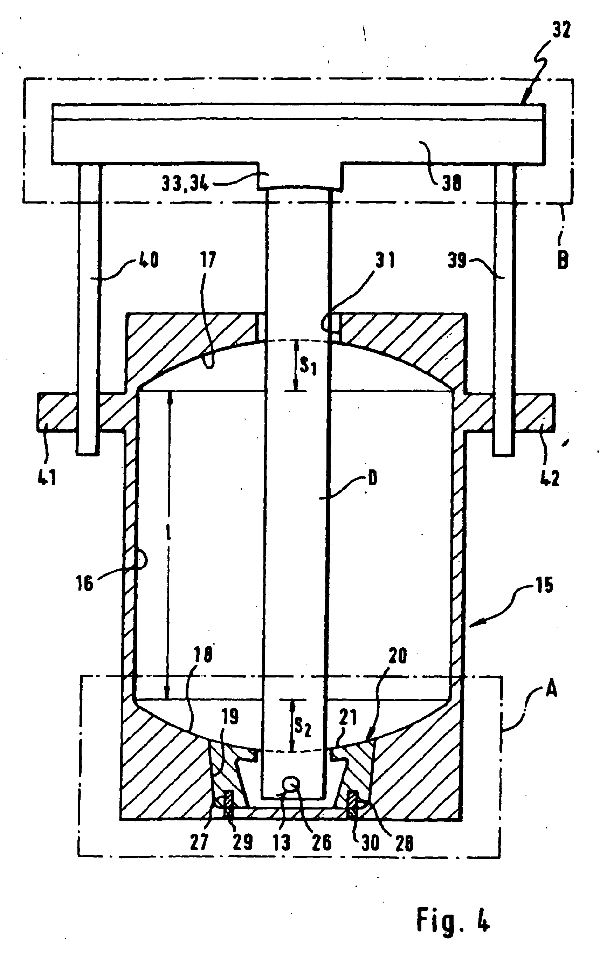

Nachfolgend wird unter Bezugnahme auf die Figuren 4 bis 7 die Aufnahmeeinheit für das Disposable beschrieben, die Bestandteil der medizinischen Behandlungsvorrichtung, beispielsweise einer Hämodialysevorrichtung ist.

Die Aufnahmeeinheit ist als formgebende Schale ausgebildet, in der das Disposable nach der Entfaltung eine genau reproduzierbare Form erhält. Die formgebende Schale 15 weist eine mittlere zylindrische Anlagefläche 16 auf, an die sich als Deckel eine nach außen gewölbte obere Anlagefläche 17 und als Boden eine nach außen gewölbte untere Anlagefläche 18 anschließt. Insofern bildet die formgebende Schale einen rotationssymmetrischen Hohlkörper, der das Disposable vollständig aufnimmt. Die obere und untere Anlagefläche 17, 18 weisen im Schnitt eine bogenförmige Kontur auf. Die Abstände zwischen den Scheitelpunkten der bogenförmigen Konturen der oberen bzw. unteren Anlagefläche 17, 18 und der zylindrischen Anlagefläche 16, d. h. die Höhe bzw. Tiefe der nach außen gewölbten Abschnitte, sind in Figur 4 mit S₁ und S₂ bezeichnet. Der zylindrische Abschnitt 16 hat die Länge 1.

Die Anlageflächen 17 und 18 können eine gleiche oder unterschiedlichen Wölbung aufweisen, wobei dies ebenfalls für die geometrische Ausgestaltung des Disposables gültig ist. So kann es zweckmäßig sein, die obere gewölbte Fläche 17 tiefer anzuordnen, damit ein möglichst eindeutig definierter Punkt für die Dialsysierflüssigkeitsentnahme vorliegt. Weiterhin kann es vorteilhaft sein, wenn die untere gewölbte Fläche 18 zur Minimierung der Bauhöhe eine flache Wölbung aufweist.

Die Schale besteht vorzugsweise aus einem wärmeisolierendem Material oder ist mit einer Wärmeisolierung versehen.

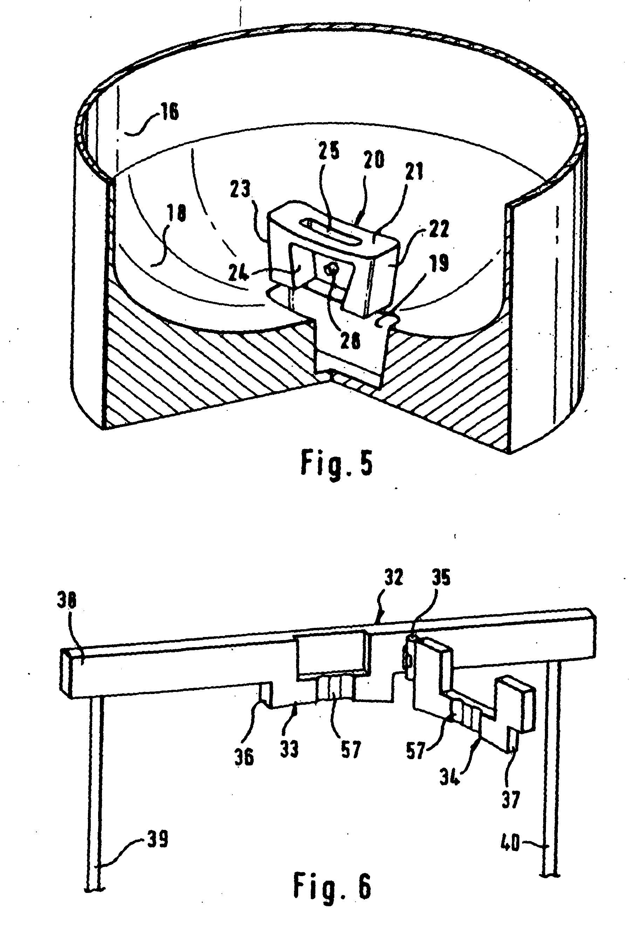

Im Zentrum der unteren Anlagefläche weist die Schale 15 eine Ausnehmung 19 auf, in die eine Fixierungseinrichtung 20 zur Befestigung des unteren Endes des Disposables D paßgenau einsetzbar ist. Die Fixierungseinrichtung 20 ist ein längliches Formstück mit einer der Kontur der unteren Anlagefläche 18 entsprechenden Oberseite 21 und zwei nach innen verlaufenden Schmalseiten 22, 23, so daß sich das Formstück beim Einsetzen in die Schale zentriert (Figur 5).

Eine der Längsseiten des Formstücks ist mit einer mittigen Ausparung 24 versehen. An seiner Oberseite weist das Formstück ein Langloch 25 auf, durch das sich das untere Ende des gefalteten Disposables D in das Formstück einschieben läßt. Zur Befestigung des Disposables befindet sich in der Aussparung 24 unterhalb des Langlochs 25 ein Stift 26. Die Fixierung des Formstücks erfolgt mittels einer Magnetkupplung, die aus zwei in das Formstück eingesetzten Magneten 27, 28 und zwei in die Schale 15 eingesetzten Magneten 29, 30 besteht. Die Fixierung kann leicht durch eine nicht näher dargestellt Mimik gelöst werden, bei der die Magneten 29, 30 über ein Fußpedal von den Magneten 27, 28 gelöst werden. Anstelle einer Magnetkupplung können aber auch andere Ver- und Entriegelungseinrichtungen vorgesehen, die manuell oder automatisch betätigt werden können.

Die Schale 15 weist im Zentrum der oberen Anlagefläche 17 eine weitere Ausnehmung 31 auf. Die Ausnehmung 31 ist ein Schlitz, der derart bemessen ist, daß die Fixierungseinrichtung 20 von oben in die Schale eingeführt werden kann. Zur Befestigung des oberen Endes des Disposables und zum Verschließen des Schlitzes ist eine weitere Fixierungseinrichtung 32 vorgesehen.

Figur 6 zeigt die Fixierungseinrichtung 32 in perspektivischer Darstellung. Die Fixierungseinrichtung 32 weist zwei Formstücke 33, 34 auf, wobei das eine Formstück 34 mit einem Scharnier 35, klappbar an dem anderen Formstück 33 befestigt ist. Beide Formstücke 33, 34 verfügen über einen unteren Abschnitt 36, 37, die paßgenau in den Schlitz 31 einsetzbar sind, wenn die Formstücke zusammengeklappt sind. Das Formstück 36 ist einstückig mit einem horizontalen Balken 38, an dem vertikale Führungsstangen 39, 40 befestigt sind, die in seitlichen Führungsstücken 41, 42 der Schale 15 in Längsrichtung verschiebbar geführt sind.

Die unteren Abschnitte 36, 37 der Formstücke 33, 34 weisen eine Kontur auf, die der Kontur der oberen Anlagefläche 17 entspricht. An den Innenseiten der beiden Formstücke 33, 34 ist eine muldenförmige Vertiefung 57 zur Aufnahme des Anschlußteils 6 des Disposables vorgesehen.

Für die medizinische Behandlung wird das untere Ende des gefalteten Disposables D durch die Ausnehmung 25 der unteren Fixierungseinrichtung 20 geschoben und das Disposable wird mittels der eine Öse bildenden Niete 13 an dem Stift 26 aufgehängt. Dann wird die untere Fixierungseinrichtung 20 an dem Disposable heruntergelassen, bis sie die untere Ausnehmung 19 der Schale verschließt. Damit ist das untere Ende des Disposables fixiert. Daraufhin wird der obere Anschlußteil 6 des Disposables in die muldenförmige Vertiefung 57 der oberen Fixierungseinrichtung 32 eingelegt und durch Zusammenklappen der Formstücke 33, 34 verklemmt. Damit ist auch das obere Ende des Disposables fixiert (Figur 4).

Beim Befüllen wird die zwischen Folienbeutel und Schale 15 befindliche Luft über eine nicht näher dargestellte Belüftungsöffnung in der Schale herausgedrückt.

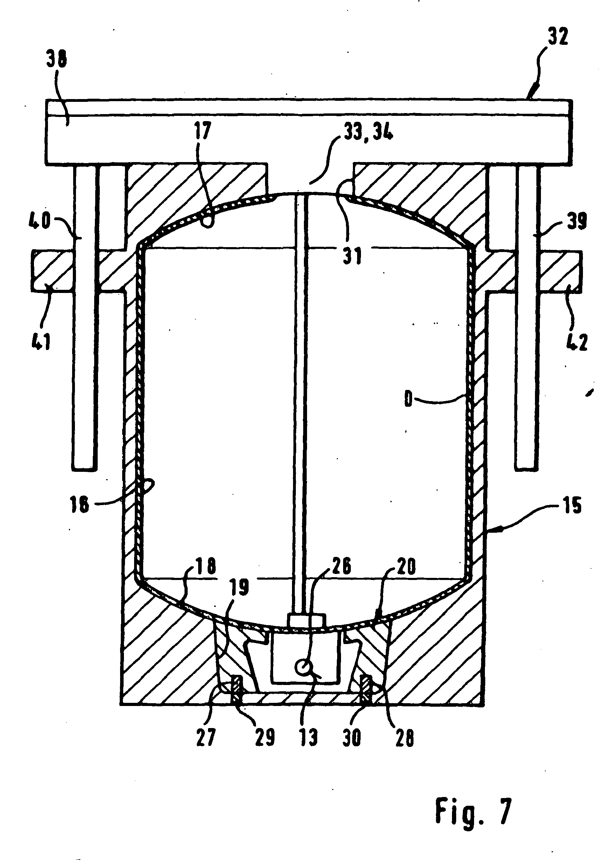

Beim Befüllen des Disposables mit Flüssigkeit entfaltet sich der Folienbeutel in der Schale. Wenn sich der Folienbeutel in der Schale entfaltet, verschiebt sich die obere Fixierungseinrichtung 32 aufgrund der Verkürzung des Folienbeutels, bis sie die obere Ausnehmung 31 vollständig verschließt (Figur 7). Der zylindrische Abschnitt des befüllten Folienbeutels liegt nun faltenfrei an dem zylindrischen Abschnitt der Schale an. Auch die nach außen gewölbten oberen und unteren Abschnitte des Folienbeutels liegen weitgehend faltenfrei an den oberen und unteren Anlageflächen der Schale an, so daß eine genau reproduzierbare Form gegeben ist.

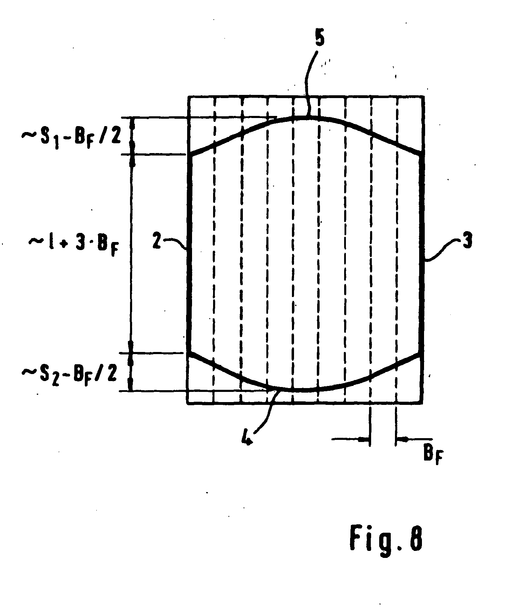

Figur 8 zeigt ein Disposable, das sich besonders einfach entfalten läßt und in seinen oberen und unteren Abschnitten zu einer besonders geringen Faltenbildung neigt. Die Bezugszeichen entsprechen den Bezugszeichen des unter Bezugnahme auf die Figuren 1 bis 3 beschriebenen Disposables. Die Länge der beiden Längsschweißnähte 2, 3 des flachliegenden Folienbeutels ist größer als die Länge 1 des zylindrischen Abschnitts der Schale 15. Sie ist um einen Betrag größer, der etwa mindestens doppelt so groß wie die Breit B_{F} der Falten des Folienbeutels ist. Eine an die Schale optimale Anpassung wird dann erreicht, wenn die Länge der Längsschweißnähte 2, 3 um einen Betrag größer ist, der etwa dreimal so groß wie die Faltenbreite B_{F} ist. Der Abstand zwischen dem Mittelpunkt der durch die gegenüberliegenden Enden der Bogenschweißnähte 4, 5 verlaufenden Geraden und den Scheitelpunkten der Bogenschweißnähte ist geringer als die Höhe bzw. Tiefe S₁, S₂ der nach außen gewölbten Abschnitte 17, 18 der Schale 15. Als optimal hat sich erwiesen, wenn der Abstand zwischen den Scheitelpunkten und den Mittelpunkten der Geraden um etwa die Hälfte der Faltenbreite B_{F} kleiner ist. Damit erhält die Bogenform einen relativ flachen Verlauf, so daß deren auslaufenden Enden auch durch gerade Schweißnähte ersetzt werden können.

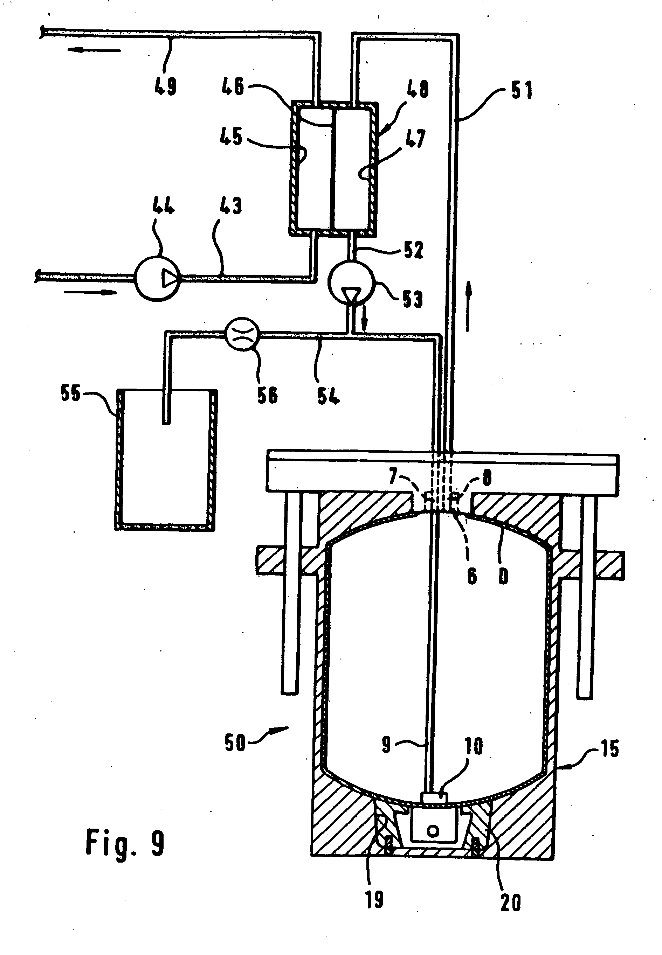

Im folgenden wird unter Bezugnahme auf Figur 9 eine Hämodialysevorrichtung beschrieben, die eine Aufnahmeeinheit gemäß der Figuren 4 bis 7 aufweist und bei der ein Disposable gemäß der Figuren 1 bis 3 bzw. 8 verwendet wird. Die Bezugszeichen von Figur 8 entsprechen den Bezugszeichen der Figuren 1 bis 8.

Das Blut des Patienten wird über eine arterielle Blutleitung 43, in die eine Blutpumpe 44 geschaltet ist, der Blutkammer 45 eines durch eine semipermeable Membran 46 in zwei Kammern 45, 47 unterteilten Dialysators 48 zugeführt und über eine venöse Blutleitung 49 zum Patient zurückgeführt. Durch die Dialysierflüssigkeitskammer 47 des Dialysators 48 fließt dazu im Gegenstrom die Dialysierflüssigkeit.

Die Dialysierflüssigkeit wird in dem Disposable gemäß der Figuren 1 bis 3 bereitgestellt, das in die Aufnahmeeinheit 50 der Hämodialysevorrichtung eingelegt wird, die anhand der Figuren 4 bis 7 beschrieben ist.

Der Einlaß der Dialysierflüssigkeitskammer 47 des Dialysators 48 ist über eine Zuführleitung 51 mit dem Anschluß 8 des Anschlußteils 6 des Disposables D verbunden, während der Auslaß der Dialysierflüssigkeitskammer über eine Rückführleitung 52, in die eine Dialysierflüssigkeitspumpe 53 geschaltet ist, mit dem Anschluß 7 des Anschlußteils 6 des Disposables verbunden ist. Von der Rückführleitung 52 zweigt stromauf des Anschlußteils 6 eine Überlaufleitung 54, ab, die in einen Behälter 55 zur Aufnahme ultrafiltrierter Flüssigkeit mündet. Die Überlaufleitung 54 kann aber auch direkt an dem Disposable angeschlossen sein. Hierzu ist es aber erforderlich, daß das Disposable über einen Anschlußteil mit drei Anschlüssen verfügt. In die Überlaufleitung 54 ist eine regelbare Drossel 56 geschaltet, um die Ultrafiltrationsrate sinnvoll begrenzen zu können.

Die Schlauchanschlüsse an das Disposable können konventionelle Anschlußstücke sein. Entscheidend ist, daß das Disposable zur Inbetriebnahme der Dialysevorrichtung schnell an das Schlauchleitungssystem anschließbar bzw. zum Austausch des Disposables wieder abnehmbar ist. Es ist auch möglich, daß das Schlauchleitungssystem einstückig mit dem Disposable ausgebildet ist.

Zur Inbetriebnahme der Hämodialysevorrichtung wird das Disposable D in die Aufnahmeeinheit 50 eingelegt und mit einer für eine Dialysebehandlung ausreichenden Menge an Dialysierflüssigkeit befüllt. Anschließend wird die Hämodialysevorrichtung in Betrieb gesetzt. Die frische Dialysierflüssigkeit strömt dann aus der oberen Hälfte des Disposables in die Dialysierflüssigkeitskammer 47 und die verbrauchte Dialysierflüssigkeit wird wieder der unteren Hälfte des Disposables zugeführt, so daß eine Vermischung von frischer und verbrauchter Dialysierflüssigkeit vermieden wird. Hierbei wird die Tatsache ausgenutzt, daß die zurückgeleitete Dialysierflüssigkeit aufgrund der im äußeren Kreislauf eintretenden Wärmeverluste stets etwas kühler ist als die frische Dialysierflüssigkeit. Auch eine wärmeisolierende Ausbildung der Aufnahmeeinheit kann dazu beitragen, daß der radiale Temperaturgradient klein gehalten wird und somit Konvektionsströmungen vermieden werden. Die optimale Schichtung von frischer und verbrauchter Dialysierflüssigkeit wird durch die annähernd zylindrische Form des entfalteten Disposables noch unterstützt.

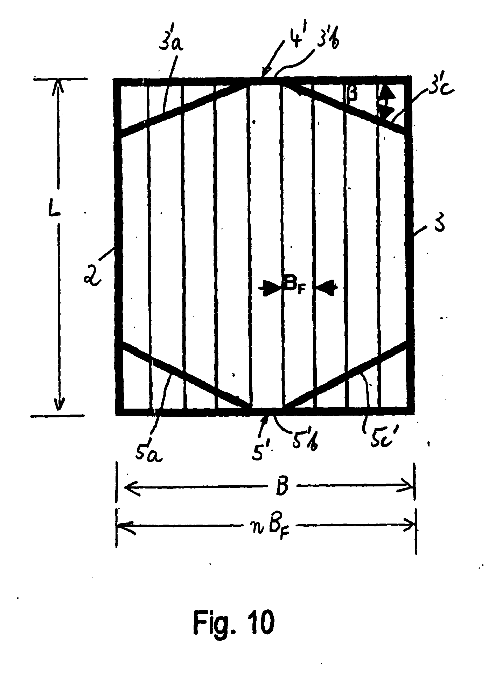

Fig. 10 zeigt ein weiteres Ausführungsbeispiel des Disposables in der Draufsicht, das für die Aufnahmeeinheit der Hämodialysevorrichtung von Fig. 9 bestimmt ist.

Diese Ausführungsform unterscheidet sich von den oben beschriebenen Ausführungsbeispielen durch die Ausbildung der nach außen weisenden Schweißnähte. Die einander entsprechenden Teile sind mit den gleichen Bezugszeichen versehen.
Das Disposable weist zwei rechteckige Folien 1,1' mit der Länge L und der Breite B auf, die deckungsgleich übereinander liegen und an den Längsseiten mit Längschweißnähten 2,3 und an den Enden mit nach außen weisenden Schweißnähten 4',5' versiegelt sind. Der flachliegende Folienbeutel ist wieder parallel zu seiner Längsachse mehrfach in n gleich große Abschnitte harmonikaartig gefaltet, wobei die Breite B_{F} der Faltabschnitte sich aus dem Quotienten der Breite B der Flachfolien und der Anzahl der Faltabschnitte berechnet. Der Anschlußteil des Disposables ist in Figur 10 nicht dargestellt.

Die nach außen weisenden Schweißnähte bestehen jeweils aus drei geraden Abschnitten 4'a, 4'b, 4'c bzw. 5'a, 5'b, 5'c, die sich produktionstechnisch einfacher herstellen lassen, als bogenförmige Schweißnähte. Beide Schweißnähte 4', 5' weisen jeweils einen mittleren Geradenabschnitt 4'b, 5'b auf, dessen Endpunkte mit den äußeren Rändern der mittleren Faltung zusammenfallen. Zu beiden Seiten des mittleren Geradenabschnitts schließen sich äußere Geradenabschnitte 4'a, 4'c bzw. 5'a, 5'c an, die sich unter einem Winkel β bis zu den Längsschweißnähten 2,3 erstrecken. Der Winkel β ist so gewählt, daß die äußeren Geradenabschnitte den gekrümmten Verlauf der Bogenschweißnähte optimal approximieren. Er berechnet sich aus der mittleren Steigung der bogenförmigen Abschweißung.

## Patentansprüche

1. Vorrichtung zur Durchführung einer medizinischen Behandlung unter Verwendung einer Flüssigkeit, insbesondere Dialysierflüssigkeit, mit einer als formgebende Schale ausgebildeten Aufnahmeeinheit (50) für ein Disposable, das ein Folienbeutel aus zwei übereinander liegenden Flachfolien oder eine Schlauchfolie mit Anschlüssen zum Zuführen und Abführen von Flüssigkeit ist, wobei der Folienbeutel bzw. die Schlauchfolie an dem in der Gebrauchslage oberen Ende mit einer ersten und an dem unteren Ende mit einer zweiten Schweißnaht verschlossen ist und der Folienbeutel bzw. die Schlauchfolie parallel der in der Gebrauchslage vertikalen Längsachse mehrfach gefaltet ist,
**dadurch gekennzeichnet, dass**
die formgebende Schale (15) eine mittlere zylindrische Anlagefläche (16) aufweist, an die sich eine nach außen gewölbte obere Anlagefläche (17) und eine nach außen gewölbte untere den Boden der formgebenden Schale (15) bildende Anlagefläche (18) anschließt,
wobei die Schale in der oberen Anlagefläche mit einer ersten Ausnehmung (31) zum Einführen des Disposables versehen ist.

2. Vorrichtung nach Anspruch 1, **dadurch gekennzeichnet, dass** die erste Ausnehmung (31) ein Schlitz im Zentrum der oberen Anlagefläche (17) der Schale (15) ist.

3. Vorrichtung nach Anspruch 1 oder 2, **dadurch gekennzeichnet, dass** die Schale (15) im Zentrum der unteren Anlagefläche (18) mit einer zweiten Ausnehmung (19) versehen ist, wobei eine Fixierungseinrichtung (20) zur lösbaren Befestigung des unteren Endes des Folienbeutels bzw. der Schlauchfolie vorgesehen ist, wobei die Fixierungseinrichtung in die zweite Ausnehmung passgenau einsetzbar ist.

4. Vorrichtung nach Anspruch 3, **dadurch gekennzeichnet, dass** die Fixierungseinrichtung (20) in der Schale arretierbar ist.

5. Vorrichtung nach einem der Ansprüche 3 oder 4, **dadurch gekennzeichnet, dass** eine zweite Fixierungseinrichtung (32) zur lösbaren Befestigung des oberen Endes des Folienbeutels bzw. der Schlauchfolie vorgesehen ist, wobei die zweite Fixierungseiririchtung in die erste Ausnehmung (31) passgenau einsetzbar ist.

6. Vorrichtung nach Anspruch 5, **dadurch gekennzeichnet, dass** die zweite Fixierungseinrichtung (32) längsverschiebbar an einer Halteeinrichtung (39 bis 42) befestigt ist.

7. Vorrichtung nach einem der Ansprüche 1 bis 6 mit einem Disposable zur Durchführung einer medizinischen Behandlung unter Verwendung einer Flüssigkeit, insbesondere zur Bereitstellung von Dialysierflüssigkeit, **dadurch gekennzeichnet, dass** das Disposable ein Folienbeutel aus zwei übereinander liegenden Flachfolien (1, 1') oder eine Schlauchfolie mit Anschlüssen (7, 8) zum Zuführen und Abführen der Flüssigkeit ist, dass der Folienbeutel bzw. die Schlauchfolie an dem in der Gebrauchslage oberen Ende mit einer ersten und an dem unteren Ende mit einer zweiten Schweißnaht (4, 5) verschlossen ist und der Folienbeutel bzw. die Schlauchfolie parallel der in der Gebrauchslage vertikalen Längsachse mehrfach gefaltet ist.

8. Vorrichtung nach Anspruche 7, **dadurch gekennzeichnet, dass** der Abstand zwischen den Enden der beiden nach außen weisenden Schweißnähte (4, 5) auf den beiden Längsseiten des flachliegenden Folienbeutels bzw. der Schlauchfolie größer ist als die Länge 1 der zylindrischen Anlagefläche der formgebenden Schale (15).

9. Vorrichtung nach Anspruch 8 oder 9, **dadurch gekennzeichnet, dass** der Abstand zwischen den Enden der beiden nach außen weisenden Schweißnähte (4, 5) auf den beiden Längsseiten des flachliegenden Folienbeutels bzw. der Schlauchfolie um einen Betrag größer ist, der mindestens doppelt so groß, vorzugsweise dreimal so groß wie die Breite B_{F} der Falten des Folienbeutels bzw. der Schlauchfolie ist.

10. Vorrichtung nach einem der Ansprüche 7 bis 9, **dadurch gekennzeichnet, dass** der Abstand zwischen dem Scheitelpunkt der ersten bzw. zweiten nach außen weisenden Schweißnaht (4, 5) und dem Mittelpunkt der durch deren Enden verlaufenden Geraden bei flachliegendem Folienbeutel bzw. Schlauchfolie kleiner als die Tiefe der gewölbten Anlageflächen (17, 18) der formgebenden Schale (15) ist.

11. Vorrichtung nach Anspruch 10, **dadurch gekennzeichnet, dass** der Abstand zwischen dem Scheitelpunkt der ersten bzw. zweiten nach außen weisenden Schweißnaht (4, 5) und dem Mittelpunkt der durch deren Enden verlaufenden Geraden bei flachliegendem Folienbeutel bzw. Schlauchfolie um einen Betrag kleiner als die Tiefe der gewölbten Anlageflächen (17, 18) der formgebenden Schale (15) ist, der etwa halb so groß wie die Breite B_{F} der Falten des Folienbeutels bzw. der Schlauchfolie ist.

12. Vorrichtung nach einem der Ansprüche 7 bis 11, **dadurch gekennzeichnet, dass** die erste und zweite Schweißnaht (4,5) einen nach außen weisenden Verlauf haben.

13. Vorrichtung nach Anspruch 12, **dadurch gekennzeichnet, dass** die erste und zweite Schweißnaht (4, 5) bogenförmig sind.

14. Vorrichtung nach Anspruch 12, **dadurch gekennzeichnet, dass** die erste und zweite Schweißnaht (4,5) jeweils mehrere gerade Abschnitte aufweist.

15. Vorrichtung nach einem der Ansprüche 7 bis 14, **dadurch gekennzeichnet, dass** der Folienbeutel bzw. die Schlauchfolie harmonikaartig gefaltet ist.

16. Vorrichtung nach einem der Ansprüche 7 bis 15, **dadurch gekennzeichnet, dass** die Faltungen mit Verbindungselementen (13, 14) fixiert sind.

17. Vorrichtung nach Anspruch 16, **dadurch gekennzeichnet, dass** die Verbindungselemente (13, 14) an den Folienenden angeordnet sind.

18. Vorrichtung nach einem der Ansprüche 7 bis 17, **dadurch gekennzeichnet, dass** an der ersten Schweißnaht (5) ein Anschlussteil (6) mit der Beutelfolie des Folienbeutels bzw. der Schlauchfolie verschweißt ist, der mindestens einen Anschluss (7) zum Zuführen von Flüssigkeit und einen Anschluss (8) zum Abführen von Flüssigkeit aufweist, wobei an dem Anschluss zum Zuführen von Flüssigkeit eine sich bis zu der zweiten Schweißnaht erstreckende Schlauchleitung (9) angeschlossen ist.

19. Vorrichtung nach Anspruch 18, **dadurch gekennzeichnet, dass** an der zweiten Schweißnaht (4) ein Fixierungsstück (10) mit der Beutelfolie des Folienbeutels bzw. der Schlauchfolie verschweißt ist, an dem die Schlauchleitung (9) befestigt ist.

20. Vorrichtung nach einem der Ansprüche 7 bis 19, **dadurch gekennzeichnet, dass** der Folienbeutel bzw. die Schlauchfolie aus einem Polyethylen-Basismaterial besteht, dass einseitig mit einer Polyamid-Siegelschicht versehen ist.

## Claims

1. A device for carrying out a medical treatment using a fluid, in particular dialysing fluid, with a receiving unit (50) designed as a shape-endowing shell for a disposable, which is a film bag comprising two flat films lying one upon the other or a tubular film with connections for the supply and discharge of fluid, wherein the film bag or tubular film is closed at the - viewed in the position of use - upper end with a first and at the lower end with a second weld seam and the film bag or the tubular film is folded repeatedly parallel to the - viewed in the position of use - vertical longitudinal axis,
**characterised in that**
the shape-endowing shell (15) comprises a central cylindrical locating surface (16), which is followed by an outwardly arched upper locating surface (17) and an outwardly arched lower locating surface (18) forming the base of the shape-endowing shell (15),
wherein the shell is provided in the upper locating surface with a first cutout (31) for introducing the disposable.

2. The device according to claim 1, **characterised in that** the first cutout (31) is a slot in the centre of the upper locating surface (17) of the shell (15).

3. The device according to claim 1 or 2, **characterised in that** the shell (15) is provided with a second cutout (19) in the centre of the lower locating surface (18), wherein a fixing device (20) is provided for the detachable fastening of the lower end of the film bag or the tubular film, wherein the fixing device can be inserted into the second cutout with a precise fit.

4. The device according to claim 3, **characterised in that** the fixing device (20) can be locked in the shell.

5. The device according to any one of claims 3 or 4, **characterised in that** a second fixing device (32) is provided for the detachable fastening of the upper end of the film bag or the tubular film, wherein the second fixing device can be inserted into the first cutout (31) with a precise fit.

6. The device according to claim 5, **characterised in that** the second fixing device (32) is fastened to a holding device (39 to 42) so as to be longitudinally displaceable.

7. The device according to any one of claims 1 to 6 with a disposable for carrying out a medical treatment using a fluid, in particular for the preparation of dialysing fluid, **characterised in that** the disposable is a film bag comprising two flat films (1, 1') lying one upon the other or a tubular film with connections (7, 8) for the supply and discharge of the fluid, that the film bag or tubular film is closed at the - viewed in the position of use - upper end with a first and at the lower end with a second weld seam (4, 5) and the film bag or the tubular film is folded repeatedly parallel to the - viewed in the position of use - vertical longitudinal axis.

8. The device according to claim 7, **characterised in that** the distance between the ends of the two outwardly pointing weld seams (4, 5) on the two longitudinal sides of the flat-lying film bag or tubular film is greater than length 1 of the cylindrical locating surface of the shape-endowing shell (15).

9. The device according to claim 8 or 9, **characterised in that** the distance between the ends of the two outwardly pointing weld seams (4, 5) on the two longitudinal sides of the flat-lying film bag or tubular film is greater by an amount which is at least twice as large, preferably three times as large, as width B_{F} of the folds of the film bag or the tubular film.

10. The device according to any one of claims 7 to 9, **characterised in that** the distance between the vertex of the first and second outwardly pointing weld seam (4, 5) and the mid-point of the straight lines running through their ends with a flat-lying film bag or a tubular film is less than the depth of the arched locating surfaces (17, 18) of the shape-endowing shell (15).

11. The device according to claim 10, **characterised in that** the distance between the vertex of the first and second outwardly pointing weld seam (4, 5) and the mid-point of the straight lines running through their ends with a flat-lying film or a tubular film is less than the depth of the arched locating surfaces (17, 18) of the shape-endowing shell (15) by an amount which is half as large as width B_{F} of the folds of the film bag or the tubular film.

12. The device according to any one of claims 7 to 11, **characterised in that** the first and second weld seam (4, 5) have an outwardly pointing course.

13. The device according to claim 12, **characterised in that** the first and second weld seam (4, 5) are arc-shaped.

14. The device according to claim 12, **characterised in that** the first and second weld seam (4, 5) each comprise a plurality of straight sections.

15. The device according to any one of claims 7 to 14, **characterised in that** the film bag or the tubular film is folded harmonica-like.

16. The device according to any one of claims 7 to 15, **characterised in that** the folds are fixed with connection elements (13, 14).

17. The device according to claim 16, **characterised in that** the connection elements (13, 14) are disposed at the film ends.

18. The device according to any one of claims 7 to 17, **characterised in that** a connection part (6) is welded at the first weld seam (5) to the bag film of the film bag or the tubular film, said connection part comprising at least one connection (7) for the supply of fluid and a connection (8) for the discharge of fluid, wherein a hose line (9) extending up to the second weld seam is connected to the connection for the supply of fluid.

19. The device according to claim 18, **characterised in that** a fixing piece (10) is welded at the second weld seam (4) to the bag film of the film bag or the tubular film, to which fixing piece the hose line (9) is fastened.

20. The device according to any one of claims 7 to 19, **characterised in that** the film bag or the tubular film is made from a polyethylene base material, which is provided on one side with a polyamide sealing layer.

## Revendications

1. Dispositif pour l'application d'un traitement médical recourant à un liquide, en particulier un liquide de dialyse, avec une unité de réception (50) réalisée comme coque qui confère sa forme à un objet jetable consistant en une poche composée de deux films plats superposés ou d'une feuille extrudée en gaine avec des raccords pour l'admission et l'évacuation du liquide, ladite poche ou ladite feuille extrudée en gaine étant fermée par un premier cordon de soudure à l'extrémité supérieure en position d'utilisation et par un deuxième cordon de soudure à l'extrémité inférieure, et ladite poche ou ladite feuille extrudée en gaine étant pliée plusieurs fois parallèlement à l'axe longitudinal vertical en position d'utilisation,
**caractérisé en ce que**
la coque (15) qui confère sa forme à l'objet présente une surface cylindrique de butée centrale (16), à laquelle sont raccordées une surface de butée supérieure (17) bombée vers l'extérieur et une surface de butée (18) inférieure bombée vers l'extérieur formant le fond de la coque (15) qui confère sa forme à l'objet,
la coque étant pourvue d'un premier évidement (31) sur la surface de butée supérieure pour l'introduction de l'objet à usage unique.

2. Dispositif selon la revendication 1, **caractérisé en ce que** le premier évidement (31) est une fente au centre de la surface de butée supérieure (17) de la coque (15).

3. Dispositif selon la revendication 1 ou la revendication 2, **caractérisé en ce que** la coque (15) est pourvue d'un deuxième évidement (19) au centre de la surface de butée inférieure (18), un système de fixation (20) étant prévu pour la fixation amovible de l'extrémité inférieure de la poche ou de la feuille extrudée en gaine, ledit système de fixation pouvant être logé avec précision d'ajustement dans le deuxième évidement.

4. Dispositif selon la revendication 3, **caractérisé en ce que** le système de fixation (20) peut être bloqué dans la coque.

5. Dispositif selon l'une des revendications 3 ou 4, **caractérisé en ce qu'**un deuxième système de fixation (32) est prévu pour la fixation amovible de l'extrémité supérieure de la poche ou de la feuille extrudée en gaine, ledit deuxième système de fixation pouvant être logé avec précision d'ajustement dans le premier évidement (31).

6. Dispositif selon la revendication 5, **caractérisé en ce que** le deuxième système de fixation (32) est maintenu de manière à pouvoir être déplacé longitudinalement sur un dispositif de maintien (39 à 42).

7. Dispositif selon l'une des revendications 1 à 6 avec un objet jetable pour l'application d'un traitement médical recourant à un liquide, en particulier pour la préparation d'un liquide de dialyse, **caractérisé en ce que** l'objet jetable consiste en une poche composée de deux films plats (1,1') superposés ou d'une feuille extrudée en gaine avec des raccords (7,8) pour l'admission et l'évacuation du liquide, **en ce que** ladite poche ou ladite feuille extrudée en gaine est fermée par un premier cordon de soudure à l'extrémité supérieure en position d'utilisation et par un deuxième cordon de soudure (4,5) à l'extrémité inférieure, et **en ce que** ladite poche ou ladite feuille extrudée en gaine est pliée plusieurs fois parallèlement à l'axe longitudinal vertical en position d'utilisation.

8. Dispositif selon la revendication 7, **caractérisé en ce que** la distance entre les extrémités des deux cordons de soudure (4,5) orientés vers l'extérieur sur les deux longueurs de la poche ou de la feuille extrudée en gaine aplatie, est supérieure à la longueur 1 de la surface de butée cylindrique de la coque (15) qui confère sa forme à l'objet.

9. Dispositif selon la revendication 7 ou 8, **caractérisé en ce que** la distance entre les extrémités des deux cordons de soudure (4,5) orientés vers l'extérieur sur les deux longueurs de la poche ou de la feuille extrudée en gaine aplatie est supérieure d'une valeur au moins double, de préférence triple à la largeur B_{F} des plis de la poche ou de la feuille extrudée en gaine.

10. Dispositif selon l'une des revendications 7 à 9, **caractérisé en ce que** la distance entre le sommet du premier ou du deuxième cordon de soudure (4,5) orienté vers l'extérieur et le centre de la droite passant par ses extrémités lorsque la poche ou la feuille extrudée en gaine est aplatie, est inférieure à la profondeur de la surface de butée bombée (17,18) de la coque (15) qui confère sa forme à l'objet.

11. Dispositif selon la revendication 10, **caractérisé en ce que** la distance entre le sommet du premier ou du deuxième cordon de soudure (4,5) orienté vers l'extérieur et le centre de la droite passant par leurs extrémités lorsque la poche ou la feuille extrudée en gaine est aplatie, est inférieure à la profondeur de la surface de butée bombée (17,18) de la coque (15) qui confère sa forme à l'objet, d'une valeur équivalant sensiblement à la moitié de la largeur B_{F} des plis de la poche ou de la feuille extrudée en gaine.

12. Dispositif selon l'une des revendications 7 à 11, **caractérisé en ce que** le premier et le deuxième cordon de soudure (4,5) présentent un tracé orienté vers l'extérieur.

13. Dispositif selon la revendication 12, **caractérisé en ce que** le premier et le deuxième cordon de soudure (4,5) sont en forme d'arc.

14. Dispositif selon la revendication 12, **caractérisé en ce que** le premier et le deuxième cordon de soudure (4,5) comportent plusieurs tronçons droits chacun.

15. Dispositif selon l'une des revendications 7 à 14, **caractérisé en ce que** la poche ou la feuille extrudée en gaine est pliée en accordéon.

16. Dispositif selon l'une des revendications 7 à 15, **caractérisé en ce que** les plis sont fixés au moyen d'éléments de connexion (13, 14).

17. Dispositif selon la revendication 16, **caractérisé en ce que** les d'éléments de connexion (13,14) sont disposés aux extrémités de la feuille.

18. Dispositif selon l'une des revendications 7 à 17, **caractérisé en ce qu'**une pièce de raccord (6) est soudée au film de la poche ou de la gaine sur le premier cordon de soudure (5), ladite pièce de raccord présentant au moins un raccord (7) pour l'admission de liquide et un raccord (8) pour l'évacuation de liquide, un tuyau flexible (9) s'étendant vers le deuxième cordon de soudure étant relié au raccord d'admission de liquide.

19. Dispositif selon la revendication 18, **caractérisé en ce qu'**une pièce de fixation (10) est soudée au film de la poche ou de la gaine sur le deuxième cordon de soudure (4), le tuyau flexible (9) étant fixé sur ladite pièce de fixation.

20. Dispositif selon l'une des revendications 7 à 19, **caractérisé en ce que** la poche ou la feuille extrudée en gaine est composée d'un matériau de base en polyéthylène, pourvu d'une couche de scellage en polyamide sur une face.
